Europäisches Patentamt

(19) European Patent Office

Office européen des brevets

(11) Publication number : **0 396 387 B1**

(12) **EUROPEAN PATENT SPECIFICATION**

(45) Date of publication of patent specification :
22.12.93 Bulletin 93/51

(51) Int. Cl.⁵ : **A61K 47/48, C12N 15/62**

(21) Application number : 90304734.8

(22) Date of filing : 01.05.90

(54) **A novel antibody delivery system for biological response modifiers.**

(30) Priority : 05.05.89 US 348237

(43) Date of publication of application :
07.11.90 Bulletin 90/45

(45) Publication of the grant of the patent :
22.12.93 Bulletin 93/51

(84) Designated Contracting States :
AT BE CH DE DK ES FR GB GR IT LI LU NL SE

(56) References cited :
EP-A- 0 150 126
EP-A- 0 184 369
EP-A- 0 305 967
EP-A- 0 317 641
EP-A- 0 336 631
EP-A- 0 350 230
WO-A-85/00974
GB-A- 1 564 666
US-A- 4 590 071
PROCEEDINGS OF AMERICAN ASSOCIATION
FOR CANCER RESEARCH vol. 28, March 1987,
ATLANTA, GEORGIA page 384 ZUCKERMAN,
J.E. et al: "Preparation and biological activity
of recombinant leukocyte interferon A[rIFN
alpha A] conjugated to an antimelanoma
murine monoclonal antibody [ZME-018]."

(56) References cited :
NUCLEAR MEDICINE AND BIOLOGY vol. 16,
no. 6, 1989, EXETER GB pages 625 - 627;
SPITLER L.E. et al: "Therapy of metastatic
malignant melanoma using xomazyme mel, a
murine monoclonal anti-melanoma ricin A
chain immunotoxin."
CANCER RESEARCH vol. 45, no. 3, March
1985, USA pages 1337 - 1343; WHITE C. A.:
"Two monoclonal antibodies selective for
human mammary carcinoma"
CANCER RESEARCH, vol. 45, 1985, pp.
1214-1221, BJORN M.J. et al.
CANCER RESEARCH, vol. 48, 1988, pp.
1119-1123, Till M. et al.

(73) Proprietor : RESEARCH DEVELOPMENT
FOUNDATION
402 North Division Street
Carson City Nevada 89703 (US)

(72) Inventor : Rosenblum, Michael G.
8801 North Rylander Circle
Houston, Texas 77071 (US)
Inventor : Wellen, Clyde William
2121 Kirby Drive, No. 79
Houston, Texas 77019 (US)

(74) Representative : Wilkinson, Stephen John et al
Stevens, Hewlett & Perkins 1 St. Augustine's
Place
Bristol BS1 4UD (GB)

EP 0 396 387 B1

## Description

The present invention relates generally to the field of immunoconjugates and, more particularly, to the use of immunoconjugates to deliver biological response modifiers to target tissue sites. The invention also relates to the treatment of cancer with conjugates of monoclonal antibodies (MoAbs) and biological response modifiers such as Tumor Necrosis Factor (TNF).

Biological response modifiers exhibit a variety of effects upon a number of cell types. Mammalian cells produce a constellation of lymphokines and cytokines to maintain homeostasis at the cellular level. In addition, in response to a variety of stimuli, mammalian cells can produce and secrete a host of protein products. At physiologic or in pharmacologic concentrations, biological response modifiers such as IFN-$\alpha$, IFN-$\beta$, IFN-$\gamma$, IL-1 - IL-7, TNF-$\alpha$ and TNF-$\beta$ can all have potent cytotoxic effects. Tumor Necrosis Factor (TNF) is one of a number of biologic response modifiers which have pleotrophic effects. The term TNF was derived from the initial observation that TNF caused necrosis of Meth A murine sarcoma. The anti-tumor effects of TNF have been demonstrated on a number of animal and human tumor cells in vitro and in vivo. Recombinant human TNF has been shown to cause tumor necrosis of sarcoma, adenocarcinomas, and melanomas. TNF has divergent effects on cell growth, causing antigrowth effects on some cell lines, no effect (i.e., less than 5% cytostasis or cytotoxicity) on other lines, and growth enhancement of other cells. TNF may also increase bone-resorption and enhance pro-coagulant activity of endothelial cells. The mechanism(s) of these pleotrophic effects is not completely understood. While IL-1, another biological response modifier, shares some biological properties with TNF, IL-1 has many properties which differ from those of TNF.

However, in order for a biological response modifier to exert an effect on a cell, the biological response modifier must reach the target cell at a concentration sufficient to cause the desired effect.

Upon the recent discovery of biological response modifiers and the elucidation of the plethora of effects caused by them, great expectations were generated that a cure for hard to treat diseases such as cancers was imminent. This, however, has not proven to be the case. One hindrance may be the inability to supply a sufficient quantity of the biological response modifier to the target tissue or cell to effect its action. When drugs or other cytoactive agents are administered to an individual, many, if not all, are diluted in the host body and are, to a certain extent, metabolized by the host tissues. Thus, in many cases these cytoactive agents must be administered in much higher amounts than are necessary to achieve the desired effects in order to account for the dilution, absorption, and metabolism by non-target tissues.

Cancer is one of the leading causes of mortality and morbidity in the Western world. There are many different types of cancer, each with its own characteristics. However, cancers share at least one characteristic in common, they involve defects in the cellular growth regulatory process. Breast cancer and cervical cancer are two of the leading causes of death from malignancy in women in the Western world. Melanoma is a highly metastatic disease affecting both sexes and is almost uniformly fatal within five years of diagnosis. Surgical removal of localized malignancies has proven effective only when the disease has not spread beyond the primary lesion. Once the disease has spread, the surgical procedures must be supplemented with other more general procedures to eradicate the diseased or malignant cells. Most of the commonly utilized alternative therapeutic modalities such as irradiation or chemotherapy do not confine their effects solely to the tumor cells and, although they have a proportionally greater destructive effect on malignant cells, often affect normal cells to some extent.

Many tumors or cancer cells express membrane-bound or cytoplasmic antigens or antigenic determinants which are either expressed very weakly or not at all by normal cells. Some tumor cells express antigens also found in or on embryonic cell types but are not expressed by normal cells of a mature animal. These abnormally expressed antigens are known as tumor-associated antigens (TAA). The TAA are specific in that while a particular antigen may be expressed by more than one tumor, it is usually expressed by all or most cells of the particular tumors which express it. A tumor cell may express one or more TAA. These tumor-associated antigens may be expressed on the surface of the cell (cell surface antigen), may be secreted by the tumor cell (secreted antigens) or may remain inside the cell (intracellular antigen). While membrane-bound or cell-surface antigens are believed to play a major role in the interaction between tumor cells and the host's immune system, cytoplasmic antigens are also useful for monitoring neoplasia since these antigens are often shed in large amounts by the tumor cells.

The presence of these tumor-associated antigens has been utilized to detect, diagnose and localize tumors in animals and man. In some cases, the presence of tumor-associated antigens has allowed the targeting of specific drugs and treatment means specifically to the tumor cells.

Antibodies are proteins normally produced by the immune system of an animal in response to foreign antigens or antigenic determinants. Antibodies bind to the specific antigen to which they are directed. Monoclonal antibodies (MoAbs) directed to specific antigens or antigenic determinants may be prepared in large quantities. Monoclonal antibodies to tumor associated antigens localize in tumors after systemic administration to patients with cancer. This powerful property has been successfully utilized to carry drugs, toxins or radioisotopes directly to tumors.

One method of targeting chemotherapeutic agents to tumor cells and to diminishing their effects on normal cells has been made possible with the development of antibodies and, more preferably, MoAbs directed against antigens on the tumor cells which do not occur on normal cells.

Antibodies, coupled to drugs, have been used as a delivery system by which the drug is targeted to a specific tumor cell type against which the antibody is directed. Antibodies may also be coupled to toxins and thus act as a delivery system to target the toxins directly to specific tumor cells. A particular advantage of the use of an antibody coupled to a drug or toxin as a delivery system to target the drug or toxin to the specific tumor is the ability to concentrate the drug or compound at the desired site. Without a specific targeting delivery system, compound administered to an individual will be dispersed throughout the entire host body and, therefore, the concentration reaching the desired target site will be much diluted. Use of an antibody targeting system will cause the administered compound attached or conjugated to the antibody to be bound to, or brought in close proximity to, the cells at the targeted site. Since the antibody-conjugated to the administered compound binds to the targeted cells at a higher rate than to other (i.e., normal cells), the compound is concentrated at the target site.

The linking of cytotoxic agents to antibodies to make "immunotoxins" has been reported. Of particular interest have been immunotoxins of monoclonal antibodies conjugated to the enzymatically active portions (A chains) of toxins of bacterial or plant origin such as Ricin or Abrin. Nevelle and York, Immunol. Rev. (1982) 62: 75-91; Ross et al., European J. Biochem. (1980) 104; Vitteta et al., Immunol. Rev. (1982) 62: 158-183; Ross et al., Cancer Res. (1982) 42: 457-464; Trowbridge and Domingo Nature (Cond.) (1981) 294: 171-173. Immunotoxins have been prepared by conjugating MoAbs with toxins or fragments of toxins derived from plants. Gelonin and ricin are among the most active plant derived toxins in inhibiting protein synthesis.

The conjugation of gelonin to antibodies has been described in EP-A-0350230 and the use of gelonin conjugated immunotoxins has been reported for the treatment of several types of cancer.

WO 8500974 discloses complexes of alpha-interferon with a monoclonal antibody which increase the serum half-life of interferon administered as the complex over that of the interferon administered alone.

Zuckerman, J E et al., Proceedings of AACR, vol. 28, 1987 (1522) discloses the coupling of interferon to monoclonal antibody ZME-018 using SPDP.

EP-A-0305967 discloses different methods for conjugating cytokines with antibodies.

Although antibodies have been used as delivery systems for toxic moieties of plant toxins and other cytotoxic drugs, conjugation of antibodies to biological response modifiers such as tumor necrosis factor and the use of such conjugates as specific delivery systems to target tissues or cells has not heretofore been possible.

The present invention is directed to a novel antibody delivery system for biological response modifiers. The present invention provides a composition of matter comprising a conjugate of an antibody directed to the 15A8 breast carcinoma antigen or the ZME-018 melanoma antigen and a modified, cytotoxic biological response modifier moiety selected from the group consisting of TNF-α, TNF-β and IL-1, said moiety being conjugated to said antibody by means of a bifunctional protein coupling agent. Preferably, the antibody is covalently bound to the biological response modifier. In one embodiment, the immunoconjugate comprises an antibody, preferably the 15A8 or ZME-018 monoclonal antibody, and TNF-α, TNF-β or IL-1 which are covalently coupled. Alternatively, the immunoconjugate may be a fusion protein prepared by genetic engineering methods known to those in the art. Such a fusion protein would contain the antigen recognition site of an antibody molecule and the cytotoxic moiety of the biological response modifier.

Since normal, as well as tumor cells, have surface receptors for biological response modifiers such as TNF, a delivery system for TNF to specifically target tumor cells enhances the effectiveness of treatment with TNF. In addition, a delivery system which selectively delivers biological response modifiers to cells in which they have a non-cytotoxic effect is also provided.

As a consequence of the concentration of the compound at the targeted site, the dose of antibody-conjugated compound which must be administered to achieve a biological response, for instance, cell killing, growth enhancement, or enzyme induction, may be orders of magnitude lower than that needed when unconjugated compound is administered.

Another advantage in utilizing an antibody-conjugated delivery system for chemical or biological compounds is the prolongation of the compound in the host system due to a decreased metabolism and/or slower rate of elimination of the conjugated compound by the host. Because the exposure of the host to a cytoactive compound is prolonged due to the decreased rate of metabolism and/or elimination, lower doses of the cytoactive compound may be administered while achieving the same magnitude of biological effects previously obtained only with higher doses. The mechanisms of metabolism and elimination of bioactive compounds is generally compound specific and is rarely totally understood. The rate of metabolism and/or elimination of almost all, if not all, bioactive compounds is slower when the bioactive compound is coupled to an antibody.

In one embodiment, the invention provides a cytotoxic composition which selectively binds to and kills tumor cells. These target tumor cells may be of any tumor which has or produces an antigenic marker in amounts greater than that found in or on normal cells. While preferably the antigenic marker or TAA is a cell surface antigen, the present invention is equally applicable to tumor cells which produce an intracellular antigen in amounts greater than normally produced by normal cells. It is known that many tumors produce intracellular antigens and either secrete them or release these antigens when the tumor becomes necrotic. The immunoconjugate of the present invention will also localize to tumor sites in which these intracellular antigens occur at a higher concentration than in normal tissue.

In another embodiment, the invention provides cytotoxic compositions which selectively bind to and are cytotoxic for or cytostatic for human breast cancer cells, cervical carcinoma cells, and melanoma cells.

Applications of the compositions of this invention include its use in a method of killing human breast cancer cells, cervical carcinoma cells, melanoma cells or other tumor cells expressing tumor associated antigen by contacting the cells with a cytocidally effective amount of the immunotoxin composition of the present invention.

The immunoconjugate of the present invention may be used to deliver a cytotoxic immunoconjugate to breast tumor cells which express the 15A8 antigen (the antigen recognized by the monoclonal antibodies disclosed and claimed in US-A-5 032 521.

In yet another embodiment the present invention provides an immunoconjugate that binds to and is cytotoxic or cytostatic for melanoma tumor cells. Melanoma cells also express several TAA. Wilson et al. 1981 Int. J. Cancer 28:293 have described two such melanoma antigens and monoclonal antibodies made thereto. One of the antibodies (225.28S) discussed by Wilson recognizes a melanoma membrane antigen. This antigen is identified herein by the designation ZME-018.

In yet another embodiment the present invention provides an immunoconjugate that binds to and is cytotoxic or cytostatic for cells which express the ZME-018 antigen or a functional equivalent thereof. The immunoconjugate of the present invention may also comprise an antibody which recognizes a cytoplasmic antigen including, but not limited to, the 465.12 antibody of Wilson which reacts with a melanoma cytoplasmic antigen.

It is an object of the invention to provide conjugates of antibodies with biological response modifiers.

It is another object of the present invention to provide a composition of matter comprising a conjugate of an antibody directed toward a tumor associated antigen and a biological response modifier moiety.

It is another object of the present invention to provide a composition comprising a recombinantly produced compound comprising an antibody moiety and a biological response modifier, which biological response modifier is a cytotoxic moiety of said biological response modifier.

It is another object of the present invention to provide an immunoconjugate of an antibody directed to the 15A8 breast carcinoma antigen or the ZME-018 melanoma antigen with a biological response modifier moiety wherein said moiety is selected from the cytoactive site of TNF-α, TNF-β and IL-1.

Another object is to provide compositions which may be used in a method of treating proliferative cell diseases such as, for instance, cancer, comprising administration of a cytocidally effective dose of an immunoconjugate comprising an antibody or antibody moiety directed to a TAA on the target cell conjugated with the biological response modifier or cytoactive moiety thereof to an individual in need of said treatment.

Another object of the present invention is to provide compositions which may be used in a method of preventing recurrence of tumors comprising administration of TNF-conjugated to a TAA monoclonal antibody to an individual in need of said treatment.

It is a further object to provide an immunoconjugate that is a gene-fusion product recombinantly produced by fusion of a gene coding for the antigen recognition site of a monoclonal antibody with a gene coding for a biological response modifier or the cytoactive moiety thereof, such as TNF-α, TNF-β and IL-1.

It is one object of the present invention to provide a selective delivery system for biological response modifiers to specific target sites.

It is another object of the present invention to prevent the recurrence of tumor-associated antigen-bearing tumors by administration of cytocidal immunoconjugates comprising a biological response modifier and an antibody to an individual in need of such treatment. Preferably, the antibody is a monoclonal antibody and the biological response modifier is TNF.

One of the objects of the present invention is to provide a cytotoxic composition which would specifically bind to and kill tumor cells.

It is a further object of the present invention to provide a composition that would be toxic to tumor cells but would cause minimal injury to normal tissue.

It is a further object of the present invention to provide a composition comprising an antibody directed to a tumor-associated antigen such as a breast tumor associated antigen or a melanoma conjugated with a biological response modifier selected from TNF-α, TNF-β and IL-1.

It was a further object of the present invention to provide a pharmaceutical composition comprising an immunoconjugate of a biological response modifier selected from TNF-α, TNF-β and IL-1 in a pharmaceutically acceptable carrier.

Figure 1 shows a S-300 Gel Permeation Chromatograph of the ZME-TNF reaction mixture.

Figure 2 demonstrates the chromatographic profile of the pooled fractions from the S-300 chromatography after affinity chromatography.

Figure 3 demonstrates the comparison of the binding of the ZME-018-TNF conjugate and free TNF to target antigen positive A-375 cells and antigen negative T-24 cells.

Figure 4 demonstrates the cytotoxicity of the ZME-TNF immunoconjugate and TNF alone on antigen-positive human melanoma cells (AAB-527).

Figure 5 demonstrates the growth inhibition of the ZME-TNF immunoconjugate on antigen-positive A-375 cells.

Figure 6 demonstrates the growth inhibition of the MoAb15A8-TNF immunoconjugate on antigen-positive ME-180 cells.

The immunochemical derivatives of this invention comprise conjugates of an antibody directed toward a tumor associated antigen and a biological response modifier.

Biological response modifiers which may be coupled to the antibody directed toward a tumor associated antigen and used in the present invention include TNF-α, TNF-β and IL-1. These biological response modifiers have a variety of effects on tumor cells. Among these effects are increased tumor cell killing by direct action as well as increased tumor cell killing by increased host defence mediated processes. Conjugation of antibody directed toward a tumor associated antigen to these biological response modifiers will allow selective localization within tumors and, hence, improved anti-proliferative effects while suppressing non- specific effects leading to toxicity of non-target cells.

Specific antibody delivery of cytotoxins to tumors will provide protection of sensitive sites such as the liver, kidney and bone marrow from the deleterious action of the naturally occurring toxic agents. Use of antibodies- conjugated to the biological response modifiers as a delivery system allows lower dosage of the drug itself, since all toxic moieties are conjugated to antibodies which concentrate within the tumor or other target site.

Conjugates of the monoclonal antibody may be made using a variety of bifunctional protein coupling agents. Examples of such reagents are SPDP, IT, bifunctional derivatives of imidoesters such as dimethyl adipimidate.HCl, active esters such as disuccinimidyl suberate, aldehydes such as glutaraldehyde, bis-azido compounds such as bis(p-azidobenzoyl) hexanediamine, bis-diazonium derivatives such as bis-(p-diazoniumbenzoyl)-ethylenediamine, diisocyanates such as tolylene 2,6-diisocyanate, and bis-active fluorine compounds such as a 1,5-difluoro-2,4- dinitrobenzene.

The antibodies employed in the immunoconjugate are preferably monoclonal antibodies,and most preferably monoclonal antibodies directed against a specific pathological conditions, including, cancers such as breast and melanoma,. The immunoconjugate of the present invention when comprised of such non-host antigens provides a selective delivery vehicle for delivering cytotoxic or cytostatic biological response modifiers to pathogenically infected host cells.

As used herein the term "monoclonal antibody" means an antibody composition having a homogeneous antibody population. It is not intended to be limited as regards the source of the antibody or the manner in which it is made.

By way of example, breast carcinoma cells express a 22/kD antigen on their cell surface. Antibodies to this antigen have been produced. Hybridomas which secrete specific monoclonal antibodies of the IgG1, IgG2a and IgG2b isotypes which recognize an epitope of this 22/kD antigen have been produced. All isotypes recognize the same epitope of the antigen. For the purpose of further example in describing this invention this epitope will be designated the 15A8 epitope. Thus, all of these antibodies are functionally equivalent. Additionally, in practicing this invention, antibodies which bind to different antigenic determinants on the same antigen, i.e., recognize different epitopes are also functionally equivalent.

Monoclonal antibodies may be made by methods known to those of skill in the art. The procedure for making the hybridoma cell cultures which produce, for instance, 15A8 MoAb, is described in detail in US-A-5 032 521 which is a continuation in part of EP-A-0-184-369 (published 11-June 1986). Briefly, mammary tumor cells were injected into BALB/c mice intraperitoneally for three weeks for a total of three to four injections. The spleens were harvested three days after the last injection and a spleen cell suspension was prepared and fused with 107 PAI mezeloma cells. The hybrid cells were selected on hypoxanthine-aminopterin thymidine (HAT) medium. Further details of the preparation of the hybridomas and characterisation of these monoclonal antibodies are provided in the examples below. However, monoclonal antibodies prepared against any TAA by any method known in the art may be used in the immunoconjugates of the present invention.

The term "Tumor associated antigen" is meant to comprise any antigen which is found in significantly higher concentrations in or on tumor cells than on normal cells.

Biological response modifiers such as TNF may be obtained from sera of intact animals, culture supernatants of lymph cells or cell lines after the animals or cells had been treated with a substance known to stimulate the proliferation of immune cells (an inducer) or by recombinant technology. The biological response modifier may be obtained from any mammalian source such as, e.g., mouse, rabbit, rat, primate, pig, and human. Preferably such proteins are obtained from a human source, and more preferably are recombinant, human proteins. Thereafter, the serum, supernatant or cell paste is harvested and assayed for biological activity toward a target tumor cell line.

The term "recombinant protein" refers to a protein having comparable biological activity to the native protein prepared by recombinant DNA techniques as is known in the art.

Recombinant DNA techniques are known in the art. In general, the gene coding for a specific protein, such as, e.g., interferon or TNF, is excised from its native plasmid and inserted into a cloning vector to be cloned and then into an expression vector, which is used to transform a host organism, preferably a microorganism, and most preferably E. coli. The host organism expresses the foreign protein gene under certain conditions to produce the specific protein, such as, e.g. TNF or interferon. For use in the present invention the recombinantly produced biological response modifier does not have to be identical in structure nor must it express the identical range of biological activities of the native protein, as long as it retains the activity sought to be delivered to the targeted site.

The biological activity of the biological response modifier and of the immunoconjugated biological response modifier may be measured by methods described in the art. For instance, TNF cytotoxic activity may be measured by using an L-929 fibroblast cell assay system as described in Example 2 below.

Antibody such as, e.g., 15A8G2 or ZME-018 were modified with SPDP as described in Example 5 below and then conjugated with iminothiolane modified TNF as described in Examples 3 and 6 below. The TNF-conjugated antibody was purified by column chromatography on a Sephadex® S-300 column as described in Example 7 below.

The toxicity of the TNF-conjugated antibody was determined by L-929 fibroblast assay and its antiproliferative activity was determined by in vitro tests.

The immunochemicals of the present invention may be used to kill tumor cells in vitro as well as in vivo. For instance, in clinical situations where bone marrow metastasis has occurred, the bone marrow may be cleared of tumor cells extracorporeally. This is often necessary when a tumor is radiosensitive and total body radiation is a required treatment. If the patient's own bone marrow can be cleared of all tumor cells, then it can be removed from the patient prior to radiation, cleared of tumor cells extracorporeally and returned to the patient to replace the bone marrow cells destroyed by radiation. When used to kill human breast cancer cells in vitro for example, the conjugates will typically be added to the cell culture medium at a concentration of at least about 10 nM. The formulation and mode of administration for in vitro use are not critical. Aqueous formulations that are compatible with the culture or perfusion medium will normally be used. Cytotoxicity may be read by conventional techniques to determine the presence or degree of breast cancer cells remaining.

Administration of the immunoconjugates of the present invention to an individual who has been diagnosed as having a tumor with a specific antigenic determinant will allow targeting and concentration of the cytotoxic agent at the site where it is needed to kill the tumor cells. By so targeting the cytotoxic agents, nonspecific toxicity to other organs, tissues and cells will be eliminated or decreased.

When used in vivo for therapy, the immunotoxins are administered to the patient in therapeutically effective amounts (i.e., amounts that eliminate or reduce the patient's tumor burden). They will normally be administered parenterally, either intravenously or intraperitoneally. The dose and dosage regimen will depend upon the nature of the cancer (primary or metastatic) and its population, the characteristics of the particular immunoconjugate, e.g., its therapeutic index, the patient, and the patient's history. The amount of immunotoxin administered will typically be in the range of about 0.1 to about 10 mg/kg of patient weight. For parenteral administration, the immunoconjugates will be formulated in a unit dosage injectable form (solution, suspension, emulsion) in association with a pharmaceutically acceptable parenteral vehicle. Such vehicles are inherently nontoxic and nontherapeutic. Examples of such vehicles are water, saline, Ringer's solution, dextrose solution, and 5% human serum albumin. Nonaqueous vehicles such as fixed oils and ethyl oleate may also be used. Liposomes may also be used as carriers. The vehicle may contain minor amounts of additives such as substances that enhance isotonicity and chemical stability, e.g., buffers and preservatives. The immunoconjugate will typically be formulated in such vehicles at concentrations of about 0.1 mg/ml to 10 mg/ml.

The immunoconjugate of the present invention is then formulated in a non-toxic, inert, pharmaceutically acceptable carrier medium, preferably at a pH of about 3 to 8, more preferably 6-8.

The dosage level of immunoconjugate will depend on the in vivo efficacy data obtained after preclinical testing and will depend mainly upon the particular biological response modifier used and whether it is used alone or in combination with other drugs or biological response modifiers.

The pharmaceutical preparation as described above is suitable for parenteral administration to humans or other mammals in therapeutically effective amounts (i.e., amounts which eliminate or reduce the patient's pathological condition) to provide therapy thereto, the type of therapy being dependent on the specific biological response modifier conjugated to the antibody delivery system.

Antibody-conjugated Biological Response Modifiers such as TNF may be administered in combination with interferon, preferably human β-interferon (IFN-β). IFN-β refers to immune interferon having comparable biological activity to native interferon. Preferably the interferon is prepared by recombinant technology.

The following examples provide a detailed description of the preparation, characterization, and use of the immunoconjugate of this invention.

## Example 1

## Purification of TNF

TNF may be purified by techniques known to these in the art. For instance, Aggarwal et al. describes the purification of TNF from various sources including several cell lines of monocytic origin. Aggarwal (1986) Methods of Enzymology 116:448.

This method may also be utilized to purify TNF from other sources.

TNF is preferably obtained by recombinant technology known to those of skill in the art. Such a preparation is, for example, described in detail in U. S. Patent No. 4,677,063 and European Publication EP 186,214. The TNF preparation used to obtain the following data depicted in the following Examples was obtained from Genentech Corp., South San Francisco, California.

The human recombinant DNA derived material was purified to homogeneity from extracts of E. coli.

TNF migrated as a single band with an approximate molecular weight of 18,000 daltons.

## Example 2

## Assay of TNF Cytotoxic Activity

The TNF cytotoxic activity was monitored utilizing the following assay on L-929 cells. Forty thousand murine L-929 fibroblasts in MEM media containing 10% FCS were added to each well of a 96 well plate and incubated 24 hrs at 37°C (5% $CO_2$). Cells were then treated with various amounts of either TNF or TNF-antibody conjugate in medium containing 0.5 g/ml Actinomycin-D for 24 hrs at 37°C (5% $CO_2$). The cells were washed with phosphate buffered saline (pH 7.2) (PBS) and viable cells were stained with crystal violet. The plates were read at 590 nm to determine viable cell number.

TNF with a specific activity no lower that $1 \times 10^7$ U/mg was used for conjugation with the antibodies. A unit of TNF activity is the amount of TNF protein which causes 50% inhibition of L-929 cell growth.

## Example 3

## Modification of TNF With Iminothiolane

TNF in phosphate buffered saline was concentrated to approximately 2 milligrams/ml in a Centricon 10 microconcentrator. Triethanolamine hydrochloride (TEA/HCl), pH 8.0 and EDTA were added to a final concentration of 60mM TEA/HCl and 1mM EDTA pH 8.0. 2-Iminothiolane stock solution (20mM) was added to a final concentration of 1 mM and the sample was incubated for 90 minutes at 4°C. under a stream of nitrogen gas.

Excess iminothiolane (IT) was removed by gel filtration on a column of Sephadex® G-25 (1 x 24cm) pre-equilibrated with 5 mM bis-tris/acetate buffer, pH 5.8 containing 50 mM NaCl and 1 mM EDTA. Fractions were analyzed for protein content in microtiter plates using the Bradford dye binding assay. Briefly, forty microliters of sample, 100 μl of phosphate buffered saline (PBS) and 40 μl of dye concentrate were added to each well. Absorbance at 600nm was read on a Dynatech Microelisa Autoreader. TNF elutes at the void volume (about fractions 14-20). These fractions are pooled and concentrated by use of a Centricon$^R$-10 microconcentrator.

## Example 4

## Preparation and Characterization of Monoclonal Antibody to 15A8 Breast Cancer Antigen and to Melanoma Antigen ZME-018

These monoclonal antibodies may be made by methods known to those of skill in the art. The procedure for making the hybridoma cell cultures which produce the 15A8 antibodies is described in detail in US-A-5 032 521 which is a continuation in part of EP-A-184-369 (published 11 June 1986). Briefly, mammary tumor cells (Soule, et al, JNCI, 51: 1409-1413 (1973) ATCC Accession No. TB-22) were injected into BALB/c mice intraperitoneally every three weeks for a total of three to four injections. The spleens were harvested three days after the last injection and a spleen cell suspension was prepared and washed by two centrifugations (800 x g) in Dulbecco's modified Eagles medium. One hundred and eight immunized mouse spleen cells and 107 PAI myeloma cells obtained from Dr. Theo Staehlin, Basel, Switzerland, J. Stocker, Research Disclosure, 21713, 155-157 (1982) were resuspended for fusion in a 45% solution (v/v) of polyethylene glycol 1500. The hybrid cells were selected on hypoxanthine-amenopterin-thymidine (HAT) medium.

Clones of the hybridoma were grown in vitro according to known tissue culture techniques such as is described by Cotten, et al., Eur. J. Immunol. 3:136 (1973). Hybridomas producing antibodies which reacted with MCF-7 and/or MDA-157 cells but not human foreskin fibroblast cells were further characterized. The antibodies produced by the 15A8 cell line and hybridomas-producing functionally equivalent antibodies reacted with the 15A8 antigen on MCF-7 cells. They also reacted with 28/31 randomly obtained human mammary carcinomas tested, and exhibited a weaker reaction with normal human epithelial cells of breast, renal proximal tubule, bladder skin, esophagus and salivary gland, but cells of substantially no other normal tissue, and was unreactive with 14 of 18 other malignant tissues tested. The 15A8 antibody also reacted with all fibrocystic diseases, normal mammary epithelium, a number of adenocarcinomas. It did not react with mesotheliomas. The 15A8 antibody also crossreacts with cervical, colon and prostrate carcinomas.

Representative hybridoma cultures whose cells secrete antibody of the same idiotype, i.e., all recognize the 15A8 epitope, have been deposited at the American Type Culture Collection of 12301 Parklawn Drive, Rockville, Maryland 20852 ("ATCC") and have been assigned the accession numbers HB-8655 (for 15A8), HB-9344 (for 15A8 G2a) and HB-9345 (for 15A8 G2b).

As used herein with respect to the exemplified murine monoclonal anti-human breast cancer antibodies, the term "functional equivalent" means a monoclonal antibody that: (1)/crossblocks an exemplified monoclonal antibody; (b)/binds selectively to cells expressing the 15A8 antigen such as human breast cancer cells; (c)/has a G or M isotype; (d)/binds to the 15A8 antigen as determined by immunoprecipitation or sandwich immunoassay; and (e)/when conjugated to TNF, exhibits a tissue culture inhibitory dose (TCID) of at least 50% against at least one of the MCF-7, ME-180, BT-20, or A431 cell lines when used at a dose between 50 and 100 units per ml.

Monoclonal antibodies which bind to melanoma cells were similarly prepared. The details of the preparation of monoclonal antibodies directed to cell surface and cytoplasmic melanoma antigens is described in detail in Wilson et al. Int. J. Cancer (1981) 28:293, incorporated herein by reference.

Example 5

Modification of Monoclonal Antibody 15A8 or ZME-018 With SPDP

N-succinimidyl 3-(2-pyridyldithio) (propionate) (SPDP) in dimethylformamide was prepared as a stock solution of 3 mg/ml in dry dimethylformamide. Since the crystalline SPDP can undergo hydrolysis, the actual concentration of chemically reactive crosslinker was determined by spectrophotometric methods by analyzing the absorbance at 260-nm in a dual-beam spectrophotometer. The concentration of SPDP stock is calculated from the following equation:

$$\frac{\text{Change in absorbance (260nm)}}{0.02 \times 10^3 \ \text{ml/mmol}} \times \frac{(3.01)}{0.01} = \text{mmoles/ml/SPDP}$$

One milligram of monoclonal antibody, for instance, MoAb 15A8 or MoAb ZME-018 in 0.5 ml of PBS was added to a glass tube. SPDP stock solution was slowly added at a 5-fold molar excess to the tube, mixing constantly. The mixture was incubated for 30 minutes at room temperature, mixing every 5 minutes during the incubation period.

Excess unreacted SPDP was removed by gel filtration chromatography on a Sephadex® G-25 column (1 x 24cm) pre-equilibrated with PBS. Fractions (0.5 ml) were collected during the PBS elution and were analyzed for protein content by the Bradford dye method. Antibody eluted in the void volume (approximately fractions 14-20). These fractions were pooled and the protein concentrated in a Centricon-30 microconcentrator. The Centricon retentate was washed with 100 mM sodium phosphate buffer, pH 7.0 containing EDTA (0.5 mM). The antibody was concentrated to a final volume of approximately 0.5-0.75 ml.

Example 6

Conjugation of SPDP-Modified Monoclonal Antibody With Iminothiolane Modified TNF

Antibody I5A8 and antibody ZME-018 were conjugated to TNF using N-succinimidyl-3-(2-pyridyldithio) propionate (SPDP) and/or iminothiolane (IT) as a coupling agent. The conjugates were tested against Me-180, and AAB-527 cells in a 72-hour tissue culture assay. The antibody conjugates exhibited acceptable antiproliferative activity (TCID 50% of less than 10 units/ml) against both of these cell lines.

Monoclonal antibody 15A8 or ZME-018 modified as described in Example 4 was mixed with an equal weight of TNF modified as in Example 3. This proportion corresponded to a 5-fold molar excess of TNF as compared to antibody. The pH of the mixture was adjusted to 7.0 by the addition of 0.05 M/TEA/HCl buffer pH 8.0 and the mixture was incubated for 20 hours at 4°C under nitrogen. Iodoacetamide (0.1M) was added to a final concentration of 2 mM to block any remaining free sulfhydryl groups and incubation was continued for an additional hour at about 25°C. The reaction mixture was stored at 4°C. until purification by gel filtration.

Example 7

Purification of TNF-Monoclonal Antibody Complexes

Non-conjugated TNF was removed from the reaction mixtures of Example 6 by gel filtration on a Sephadex® S-300 column (2.5 x 50 cm) pre-equilibrated with PBS.

Reaction mixtures from Example 6 containing TNF conjugated to MoAb ZME were concentrated to approximately 1 ml with a Centricon 30 microconcentrator before loading on the Sephadex column. The column was washed with PBS. One ml fractions were collected and 50 µl aliquots are analyzed for protein by the Bradford dye binding assay. M. Bradford, Anal. Biochem 72:248 (1976).

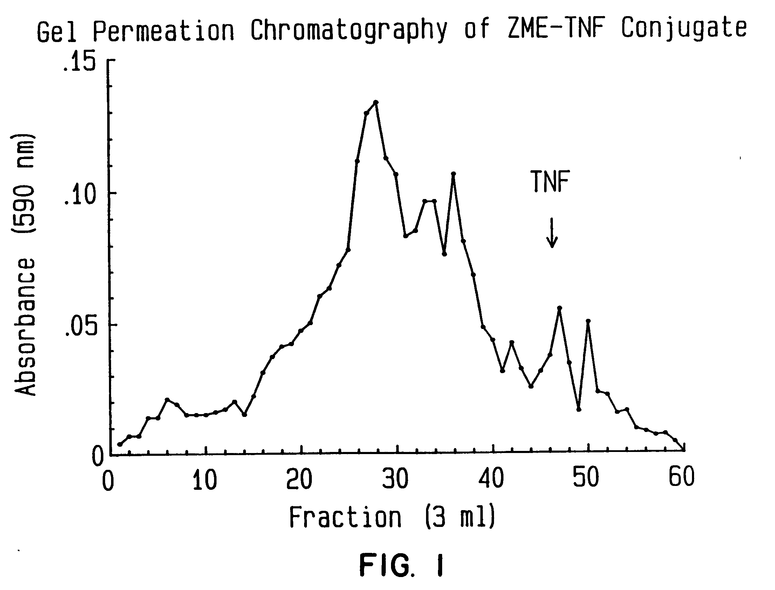

Free- and TNF-conjugated antibody eluted at about fractions 17-40 while, unconjugated TNF elutes at about fractions 46-50. Figure 1 demonstrates the elution profile of the S-300 column. Elution of free TNF standard (in fraction 46-48) is shown by arrow. Elution of unconjugated ZME antibody occurs at approximately fraction 28. After chromatography of the reaction mixture, fraction 20-40 were pooled. PAGE analysis demonstrated that these fractions contained no free TNF. This elution pattern was confirmed by electrophoresis of 50 µl aliquots on 5-20% gradient non-reducing SDS polyacrylamide gels. This analysis confirmed that the conjugated material contained from one to three molecules of TNF coupled per molecule of antibody and no free TNF.

Non-conjugated antibody was removed from the TNF-conjugated antibody by affinity chromatography using CNBr sepharose coupled to a murine anti-TNF antibody. The resin was poured into a small column (1 x 4cm) and pre-equilibrated with 10mM phosphate buffer, pH 7.2 containing 0.1M NaCl. After loading the S-300 pooled sample, the column was washed with 30 ml of the same buffer to completely elute non-conjugated antibody. TNF-conjugated antibody bound to the column.

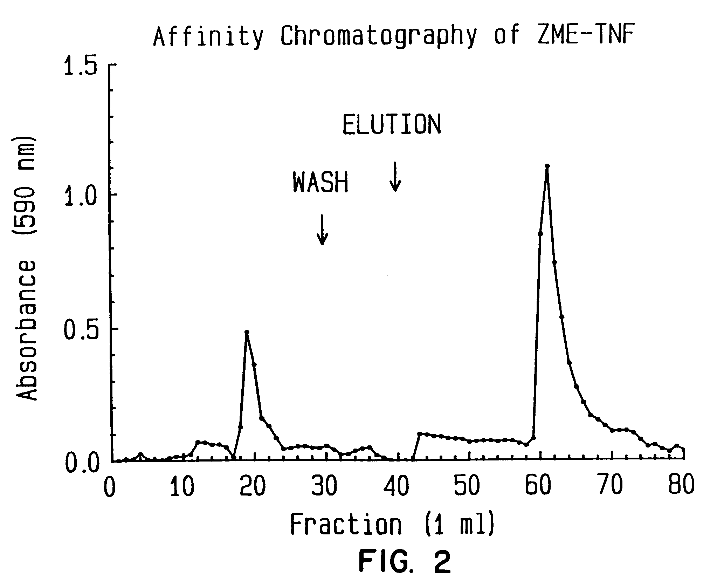

The antibody-TNF complex eluted from the column as shown in Figure 2 which depicts the elution profile of the TNF affinity column. The flow-through peak contains only free antibody while fractions 58-70, contain antibody-TNF conjugate free of unconjugated TNF or antibody. The pooled fractions from the S-300 chromatography were applied to an affinity chromatography support to which an anti-TNF murine antibody was bound. The column was washed extensively with PBS allowing free ZME antibody (Fraction 20 peak) to elute from the resin. Elution of ZME-TNF conjugate was performed by washing the column with 0.1M Na acetate buffer (pH 4.5) containing 0.15M NaCl buffer. As shown in Figure 2, the purified conjugate was eluted as a single protein peak. PAGE analysis on a 5-20% acrylamide continuous gradient non-reducing gel demonstrated that the conjugate contained ZME bound to 1, 2 and 3 TNF molecules. There was no detectable free TNF or free ZME-018 in the final product.

Protein content of the eluted fractions was determined by the Bradford dye binding assay. The protein-containing fractions were pooled and the elution pattern confirmed by electrophoresis on a 5 to 20% gradient non-reducing polyacrylamide gel.

The L-929 assay described in Example 2 was utilized to estimate the TNF activity of the essentially pure TNF-antibody complex. Both the essentially pure 15A8-TNF and the ZMF-TNF antibody conjugates are active in the L-929 assay. A 1:1000 dilution of the original sample caused approximately a 50% inhibition of L-929 cell growth. Thus, the activity of the original preparation was 1000 U/ml.

Example 8

Comparison of Binding of TNF-conjugated and Unconjugated ZME-018 Antibody to Target Cells

In order to determine whether changes in the binding characteristics of ZME to target A-375 (antigen positive) cells or T-24 cells (antigen negative) occurred by modification with TNF, cells were plated at 50,000 cells/well in a 96-well plastic plate and allowed to air-dry at room temperature. Various concentrations of ZME or ZME-TNF were added, allowed to bind for 3 h at room temperature. A standard ELISA assay was performed for detection of murine antibody.

The ability of the TNF-conjugated and unconjugated ZME-018 antibody to bind to target cells was assessed. Fifty thousand target cells (A-325) or non-target Human bladder carcinoma cells (T-24 cells) were added to each well of microtiter plate. The cells were dried on the plates overnight at 37°C. The cells were then washed with three changes of cold PBS and air dried overnight. The cell surface antigenic determinants remain antigenically active after this treatment.

After attachment of the cells, the plates were washed with Washing Buffer (9.68 Tris, 64.8 sodium chloride, 16 ml Tween 20, 800 mg thimerasol in 8 l of double distilled water). Antibody samples were diluted in Washing Buffer containing 1% Bovine serum albumin (w/v) (Diluting buffer). Fifty microliters of various concentrations ranging from 0.002 to 100 µg/ml of either conjugated or unconjugated ZME-018 antibody were added to the wells. After incubation for 1 hour at 4°C, the supernatants are removed and the wells washed twice with Washing Buffer.

Fifty microliters per well of alkaline phosphatase-conjugated goat anti-mouse IgG obtained from Bio-Rad and diluted 1:1000 (v/v) (APGAM) in Diluting Buffer was added to each well. The plates were incubated for 1 hour at 4°C and the wells washed twice with Washing Buffer. After incubation of the plates with 50 µl of Substrate Solution (80 mM citrate phosphate (pH 5.0), 1 mM ABTS substitute and 4 µl of 30% hydrogen peroxide) in the dark for 30 minutes at room temperature, 25 µl of 4 N sulfuric acid was added to each well. The absorbance at 492 nm was determined on an Elisa plate reader.

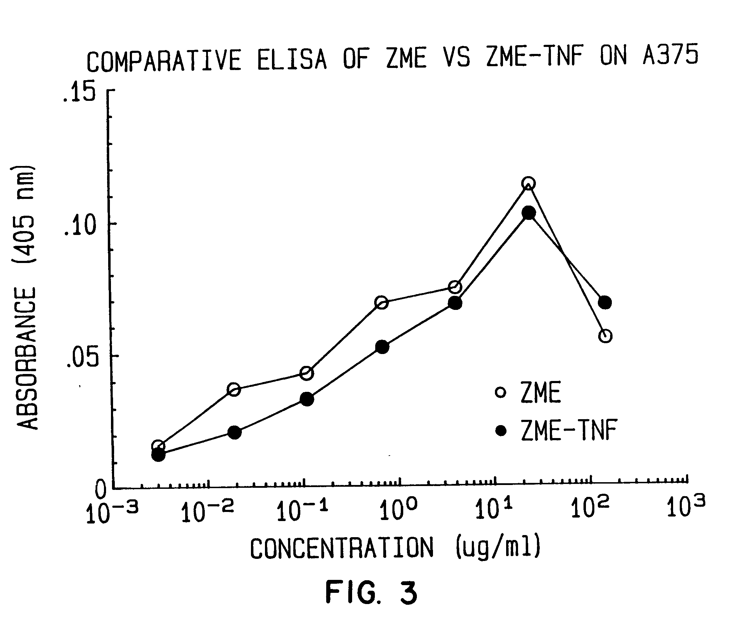

The results are shown on Figure 3. The ZME-TNF complex bound to A-375 target cells to the same extent as did native ZME antibody. Since there was no difference in the binding of the ZME-TNF or the unconjugated ZME antibody to the A-135 antigen containing target cells, the chemical conjugation procedure does not alter the affinity of the antibody for its target antigen. There was no detectable binding of either ZME or ZME-TNF complex to non-target T-24 bladder carcinoma cells.

## Example 9

### Antiproliferative Effects of TNF and TNF-15A8 or ZME-TNF Antibody Complex

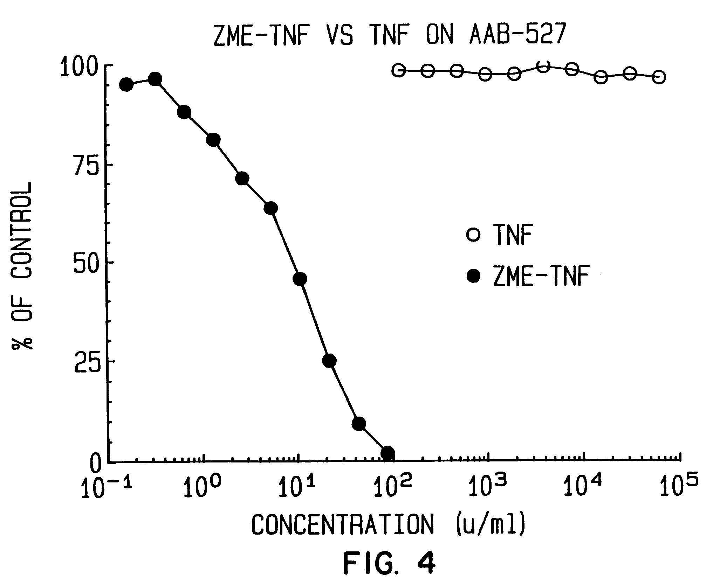

The antiproliferative effects of TNF and 15A8-TNF or ZME-TNF conjugate were assessed by plating approximately 5,000 log-phase cells/well in 96 well microtiter plate in 200 ml of appropriate tissue culture media. The cells were allowed to adhere for 24 hours at 37°C in atmosphere of 5% $CO_2$ in air. Non-targeted, antigen negative T-24 human bladder carcinoma cells, Me-180 cells antigen positive for 15A8 and A-375 human melanoma cells antigen positive for ZME-018 in log-phase were treated with various concentrations of either media alone (control), TNF 15A8-TNF conjugate or ZME-TNF conjugate and incubated at 37°C in an atmosphere of 5% $CO_2$ in air for 72 hours. The plates were washed three times with cold PBS. 50ml of methanol was added to each well and the cells lysed by repeated cycles of freezing and thawing. Protein concentrations were then determined by the Bradford dye test. Alternatively, cell numbers in each well was assessed using crystal violet stain. The absorbance of each well was determined on an ELISA reader and compared to control wells (no treatment). As shown in Figure 4, TNF alone had no cytotoxic or cytostatic effect at the concentration of TNF used (50,000 units/well). However, with the ZME-TNF conjugate, 50% inhibition was obtained with only 10 units/ml.

Cell growth inhibition was also assessed by reduction in protein concentrations or cell number of treated cells as compared to saline-treated controls. There was no inhibition of cell growth by the 15A8-TNF conjugate or the ZME-TNF T-24 carcinoma on non-targeted T-24 carcinoma cells.

There was no effect of 15A8-TNF against the T-24 non-target cell line.

Since only cells containing the 15A8 antigen on their surface were killed by the TNF 15A8 immunotoxin, this immunotoxin is an efficient method to target and kill 15A8 tumor associated antigen-containing cells while minimizing or preventing damage or injury to normal non-tumor associated antigen-bearing cells.

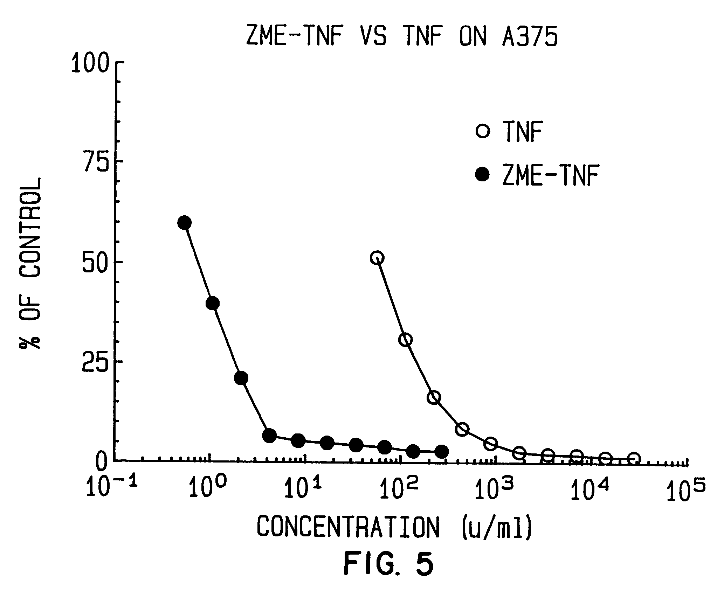

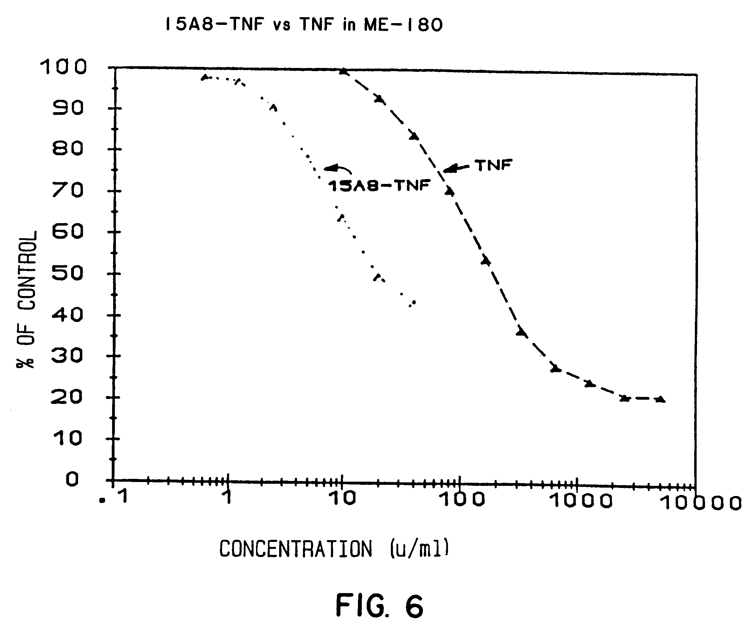

The ZME-TNF conjugate was more active than free TNF when tested on antigen-positive human melanoma (either AAB-27 or A-375) cells in culture (Figures 4 and 5). As shown in Fig. 4, TNF alone had no effect on the growth of AAB-27 cells at doses up to 50,000 U/ml. However, 50% inhibition was obtained with approximately 6 U/ml of the ZME-TNF conjugates as shown in Figure 5. A-375 target human melanoma cells were inhibited by TNF alone at doses of approximately 100 U/ml, while the ZME-TNF conjugate inhibited cells at a concentration of approximately 0.8 U/ml. Me-180 target cells were 10 fold more sensitive to the 15A8-TNF immunotoxin than to TNF alone (Figure 6).

Figure 6 demonstrates that at approximately 15 U/ml, TNF conjugated 15A8 antibody inhibited 50% of the Me-180 cells, while a concentration of 200 U/ml of the unconjugated TNF was required to achieve the same effect. There was no effect of either TNF or ZME-TNF conjugate on antigen-negative T-24 cells.

Thus, immunoconjugates of ZME and 15A8 with TNF can dramatically augment the cytotoxicity of TNF on antigen-positive cells while antigen negative cells are unaffected. In addition, in one cell line totally resistant to the growth-inhibiting effects of TNF alone (Fig. 4), cellular resistance can be overcome by antibody targeting.

One skilled in the art will readily appreciate that the present invention is well adapted to carry out the objects and obtain the ends and advantages mentioned, as well as those inherent therein. The compounds, methods, procedures and techniques described herein are presently representative of the preferred embodiments, are intended to be exemplary. Changes therein and other uses of the invention are defined by the scope of the appended claims.

## Claims

### Claims for the following Contracting States : AT, BE, CH, DE, DK, FR, GB, IT, LI, LU, NL, SE

1. A composition of matter comprising a conjugate of an antibody directed to the 15A8 breast carcinoma antigen or the ZME-018 melanoma antigen and a modified cytotoxic biological response modifier moiety selected from TNF-$\alpha$, TNF-$\beta$ and IL-1, said moiety being conjugated to said antibody by means of a bifunctional protein coupling agent.

2. A composition as claimed in claim 1, wherein said composition is a recombinantly produced compound.

3. A composition as claimed in claim 1 or claim 2, wherein said antibody is 15A8 antibody.

4. A composition as claimed in claim 1 or claim 2, wherein said antibody is ZME-018 antibody.

5. A composition as claimed in claims 2 to 4, wherein said conjugate is a gene-fusion product recombinantly produced by fusion of a gene coding for an antigen recognition site of a monoclonal antibody with a gene coding for a biological response modifier.

6. A composition comprising a human interferon and a conjugate of an antibody directed to the 15A8 breast carcinoma antigen or the ZME-018 melanoma antigen and a biological response modifier moiety selected from TNF-$\alpha$, TNF-$\beta$ and IL-1, said moiety being conjugated to said antibody by means of a bifunctional protein coupling agent.

**Claims for the following Contracting States : ES, GR**

1. A method for producing a composition of matter comprising a conjugate of an antibody directed to the 15A8 breast carcinoma antigen or the ZME-018 melanoma antigen and a modified cytotoxic biological response modifier moiety selected from TNF-$\alpha$, TNF-$\beta$ and IL-1, said moiety being conjugated to said antibody by means of a bifunctional protein coupling agent, comprising the step of conjugating said antibody with said moiety.

2. A method for producing a composition of matter comprising a conjugate of an antibody directed to the 15A8 breast carcinoma antigen or the ZME-018 melanoma antigen and a modified cytotoxic biological response modifier moiety selected from TNF-$\alpha$, TNF-$\beta$ and IL-1, said moiety being conjugated to said antibody by means of a bifunctional protein coupling agent, comprising a step involving a recombinant technique.

3. A method as claimed in claim 1 or claim 2, wherein said antibody is 15A8 antibody.

4. A method as claimed in claim 1 or claim 2, wherein said antibody is ZME-018 antibody.

5. A method as claimed in claims 2 to 4, wherein said conjugate is a gene-fusion product recombinantly produced by fusion of a gene coding for an antigen recognition site of a monoclonal antibody with a gene coding for a biological response modifier.

6. A method for producing a composition comprising a human interferon and a conjugate of an antibody directed to the 15A8 breast carcinoma antigen or the ZME-018 melanoma antigen and a biological response modifier moiety selected from TNF-$\alpha$, TNF-$\beta$ and IL-1, said moiety being conjugated to said antibody by means of a bifunctional protein coupling agent, comprising the step of conjugating said antibody with said moiety.

7. A method for producing a composition comprising a human interferon and a conjugate of an antibody directed to the 15A8 breast carcinoma antigen or the ZME-018 melanoma antigen and a biological response modifier moiety selected from TNF-$\alpha$, TNF-$\beta$ and IL-1, said moiety being conjugated to said antibody by means of a bifunctional protein coupling agent, comprising a step involving a recombinant technique.

**Patentansprüche**

**Patentansprüche für folgende Vertragsstaaten : AT, BE, CH, DE, DK, FR, GB, IT, LI, LU, NL, SE**

1. Zusammensetzung, umfassend ein Konjugat eines gegen das 15A8-Brustkarzinom-Antigen oder das ZME-018-Melanom-Antigen gerichteten Antikörpers und eines Anteils eines modifizierten cytotoxischen Modifikators des biologischen Ansprechvermögens, ausgewählt aus TNF-$\alpha$, TNF-$\beta$ und IL-1, wobei der Anteil mit dem Antikörper konjugiert ist mit Hilfe eines bifunktionellen Protein-Kupplungsmittels.

2. Zusammensetzung nach Anspruch 1, worin die Zusammensetzung eine rekombinant erzeugte Verbindung ist.

3. Zusammensetzung nach Anspruch 1 oder Anspruch 2, worin der Antikörper ein 15A8-Antikörper ist.

4. Zusammensetzung nach Anspruch 1 oder Anspruch 2, worin der Antikörper ein ZME-018-Antikörper ist.

5. Zusammensetzung nach einem der Ansprüche 2 bis 4, worin das Konjugat ein Gen-Fusions-Produkt ist, das rekombinant erzeugt wurde durch Fusion eines Gens, das eine Antigen-Erkennungsstelle eines monoklonalen Antikörpers kodiert, mit einem Gen, das einen Modifikator des biologischen Ansprechvermögens kodiert.

6. Zusammensetzung, umfassend Human-Interferon und ein Konjugat eines gegen das 15A8-Brustkarzinom-Antigen oder ZME-018-Melanom-Antigen gerichteten Antikörpers und eines Anteils eines Modifikators des biologischen Ansprechvermögens, ausgewählt aus TNF-$\alpha$, TNF-$\beta$ und IL-1, wobei der Anteil mit dem Antikörper konjugiert ist mit Hilfe eines bifunktionellen Protein-Kupplungsmittels.

**Patentansprüche für folgende Vertragsstaaten : ES, GR**

1. Verfahren zur Herstellung einer Zusammensetzung, umfassend ein Konjugat eines gegen das 15A8-Brustkarzinom-Antigen oder das ZME-018-Melanom-Antigen gerichteten Antikörpers und eines Anteils eines modifizierten cytotoxischen Modifikators des biologischen Ansprechvermögens, ausgewählt aus TNF-$\alpha$, TNF-$\beta$ und IL-1, wobei der Anteil mit dem Antikörper konjugiert ist mit Hilfe eines bifunktionellen Protein-Kupplungsmittels, umfassend die Stufe, daß man den Antikörper mit dem Anteil konjugiert.

2. Verfahren zur Herstellung einer Zusammensetzung, umfassend ein Konjugat eines gegen das 15A8-Brustkarzinom-Antigen oder ZME-018-Melanom-Antigen gerichteten Antikörpers und eines Anteils eines modifizierten cytotoxischen Modifikators des biologischen Ansprechvermögens, ausgewählt aus TNF-$\alpha$, TNF-$\beta$ und IL-1, wobei der Anteil mit dem Antikörper mit Hilfe eines bifunktionellen Protein-Kupplungsmittels konjugiert ist, umfassend eine Stufe unter Anwendung einer rekombinanten Technik.

3. Verfahren nach Anspruch 1 oder Anspruch 2, worin der Antikörper ein 15A8-Antikörper ist.

4. Verfahren nach Anspruch 1 oder Anspruch 2, worin der Antikörper ein ZME-018-Antikörper ist.

5. Verfahren nach einem der Ansprüche 2 bis 4, worin das Konjugat ein Gen-Fusions-Produkt ist, das rekombinant erzeugt wurde durch Fusion eines Gens, das eine Antigen-Erkennungsstelle eines monoklonalen Antikörpers kodiert mit einem Gen, das einen Modifikator des biologischen Ansprechvermögens kodiert.

6. Verfahren zur Herstellung einer Zusammensetzung, umfassend Human-Interferon und ein Konjugat eines gegen das 15A8-Brustkarzinom-Antigen oder ZME-018-Melanom- Antigen gerichteten Antikörpers und eines Anteils eines Modifikators des biologischen Ansprechvermögens, ausgewählt aus TNF-$\alpha$, TNF-$\beta$ und IL-1, wobei der Anteil mit dem Antikörper konjugiert ist mit Hilfe eines bifunktionellen Protein-Kupplungsmittels, umfassend die Stufe, daß man den Antikörper mit dem Anteil konjugiert.

7. Verfahren zur Herstellung einer Zusammensetzung, umfassend Human-Interferon und ein Konjugat eines gegen das 15A8-Brustkarzinom-Antigen oder ZME-018-MelanomAntigen gerichteten Antikörpers und eines Anteils eines Modifikators des biologischen Ansprechvermö- gens, ausgewählt aus TNF-$\alpha$, TNF-$\beta$ und IL-1, wobei der Anteil mit dem Antikörper mit Hilfe eines bifunktionellen Protein-Kupplungsmittels konjugiert ist, umfassend eine Stufe unter Anwendung einer rekombinanten Technik.

**Revendications**

**Revendications pour les Etats contractants suivants : AT, BE, CH, DE, DK, FR, GB, IT, LI, LU, NL, SE**

1. Composition comprenant un conjugué d'un anticorps dirigé contre l'antigène 15A8 du cancer du sein ou l'antigène ZME-018 du mélanome et un fragment de modificateur de la réponse biologique cytotoxique modifié, choisi parmi le TNF-$\alpha$, le TNF-$\beta$ et l'IL-1, ledit fragment étant conjugué audit anticorps par un agent bifonctionnel de couplage des protéines.

2. Composition selon la revendication 1, dans laquelle ladite composition est un composé produit par recombinaison.

3. Composition selon la revendication 1 ou la revendication 2, dans laquelle ledit anticorps est l'anticorps 15A8.

4. Composition selon la revendication 1 ou la revendication 2, dans laquelle ledit anticorps est l'anticorps ZME-018.

5. Composition selon les revendications 2 à 4, dans laquelle ledit conjugué est un produit de fusion de gènes, produit par recombinaison par fusion d'un gène codant pour un site de reconnaissance d'antigène d'un anticorps monoclonal avec un gène codant pour un modificateur de la réponse biologique.

6. Composition comprenant un interféron humain et un conjugué d'un anticorps dirigé contre l'antigène 15A8 du cancer du sein ou l'antigène ZME-018 du mélanome et un fragment de modificateur de la réponse biologique choisi parmi TNF-$\alpha$, TNF-$\beta$ et IL-1, ledit fragment étant conjugué audit anticorps par un agent de couplage bifonctionnel des protéines.

12

**Revendications pour les Etats contractants suivants : ES, GR**

1. Procédé pour préparer une composition comprenant un conjugué d'un anticorps dirigé contre l'antigène 15A8 du cancer du sein ou l'antigène ZME-018 du mélanome et un fragment de modificateur de la réponse biologique cytotoxique modifié, choisi parmi le TNF-$\alpha$, le TNF-$\beta$ et l'IL-1, ledit fragment étant conjugué audit anticorps par un agent bifonctionnel de couplage des protéines, comprenant l'étape de conjugaison dudit anticorps avec ledit fragment.

2. Procédé pour préparer une composition comprenant un conjugué d'un anticorps dirigé contre l'antigène 15A8 du cancer du sein ou l'antigène ZME-018 du mélanome et un fragment de modificateur de la réponse biologique cytotoxique modifié, choisi parmi le TNF-$\alpha$, le TNF-$\beta$ et l'IL-1, ledit fragment étant conjugué audit anticorps par un agent bifonctionnel de couplage des protéines, comprenant une étape faisant intervenir une technique de recombinaison.

3. Procédé selon la revendication 1 ou la revendication 2, dans lequel ledit anticorps est l'anticorps 15A8.

4. Procédé selon la revendication 1 ou la revendication 2, dans lequel ledit anticorps est l'anticorps ZME-018.

5. Prodédé selon les revendications 2 à 4, dans lequel ledit conjugué est un produit de fusion de gènes, produit par recombinaison par fusion d'un gène codant pour un site de reconnaissance d'antigène d'un anticorps monoclonal avec un gene codant pour un modificateur de la réponse biologique.

6. Procédé pour préparer une composition comprenant un interféron humain et un conjugué d'un anticorps dirigé contre l'antigène 15A8 du cancer du sein ou l'antigène ZME-018 du mélanome et un fragment de modificateur de la réponse biologique, choisi parmi TNF-$\alpha$, TNF-$\beta$ et IL-1, ledit fragment étant conjugué audit anticorps par un agent de couplage bifonctionnel des protéines, comprenant l'étape de conjugaison dudit anticorps audit fragment.

7. Procédé pour préparer une composition comprenant un interféron humain et un conjugué d'un anticorps dirigé contre l'antigène 15A8 du cancer du sein ou l'antigène ZME-018 du mélanome et un fragment de modificateur de la réponse biologique, choisi parmi TNF-$\alpha$, TNF-$\beta$ et IL-1, ledit fragment étant conjugué audit anticorps par un agent de couplage bifonctionnel des protéines, comprenant une étape faisant intervenir une technique de recombinaison.

Gel Permeation Chromatography of ZME-TNF Conjugate

FIG. 1

FIG. 2

Affinity Chromatography of ZME-TNF

FIG. 3

COMPARATIVE ELISA OF ZME VS ZME-TNF ON A375

ABSORBANCE (405 nm)

CONCENTRATION (ug/ml)

o ZME
● ZME-TNF

FIG. 4

FIG. 5

FIG. 6

15A8-TNF vs TNF in ME-180

CONCENTRATION (u/ml)

% OF CONTROL